# EUROPÄISCHE PATENTSCHRIFT

(11) **EP 0 778 031 B1**
(45) Veröffentlichungstag und Bekanntmachung des Hinweises auf die Patenterteilung: **06.10.1999**
(21) Anmeldenummer: 96118573.3
(22) Anmeldetag: 20.11.1996
(51) Int. Cl.: A61M 1/00, A61M 1/36

(54) **Ausscheiden von Luft aus lufthaltigem Blut**
Elimination of air from blood
Elimination de l'air du sang

(30) Priorität: 06.12.1995 DE 19545404
(43) Veröffentlichungstag der Anmeldung: 11.06.1997
(73) Patentinhaber: Convergenza AG, 9490 Vaduz (LI)
(72) Erfinder: Brockhoff, Alexander, 9494 Schaan (LI)
(74) Vertreter: Vetter, Ewald Otto, Dipl.-Ing.

(56) Entgegenhaltungen:
- GB-A- 2 063 108
- US-A- 4 368 118

## Beschreibung

Die Erfindung betrifft eine Vorrichtung zum Ausscheiden von Luft aus lufthaltigem Blut gemäß den unabhängigen Ansprüchen.

Solche sind aus der GB-A-2 063 108 bekannt.

Die Erfindung betrifft insbesondere das Trennen von Luft aus einem strömenden Blutstrom, welcher von einem Patienten, zum Beispiel einer Wundstelle oder Operationsstelle, oder von einem Blutbehälter oder einer Blutspendevorrichtung abgesaugt wird.

Beim Absaugen von Blut, z.B. von einem Patienten während einer Operation, wird häufig auch Luft aus der Umgebung abgesaugt. Die Luft vermischt sich mit dem Blut und führt zu einer Schädigung der Blutbestandteile. Dadurch wird eine Aufbereitung und Wiederverwendung des Blutes erschwert.

In der Praxis wird heutzutage von der Operationswunde eines Patienten Blut durch Systeme abgesaugt, welche aus einer Kanüle, einem Fördersystem in Form einer Rollerpumpe oder einer Vakuumpumpe, einem Blutrezirkulationssystem und Verbindungsleitungen bestehen. Diese bekannten Systeme traumatisieren (beschädigen) das Blut in weitem Ausmaß.

Die Ursachen für die Blut-Traumatisierung durch die bekannten Systeme sind unter anderem folgende:
1. Das aktive Absaugen von Blut von dem Operationsbereich eines Patienten hat eine intensive Vermischung der Flüssigkeitsphase (Blut) mit der Gasphase (Luft) zur Folge. Diese Vermischung findet nicht nur an und in der Absaugkanüle statt, sondern auch in den Verbindungsleitungen, und bildet einen Hauptfaktor für die Blut-Traumatisierung.
2. Um eine wirksame Absaugung des Blutes zu erreichen, erfordern die bekannten Systeme ein verhältnismäßig starkes Vakuum, welches zusätzliche Beschädigungen der Blutbestandteile verursacht.

Neuere Systeme zur Blutabsaugung sind deshalb so ausgebildet, daß sie die Gasphase von der Flüssigkeitsphase trennen können, um die Beschädigung des Blutes zu limitieren. Die bekannten Systeme sind jedoch sperrig, groß, schwer, schwierig zu bedienen und teuer in der Herstellung. Ein solches System ist beispielsweise aus der US-A-4 388 922 bekannt.

Durch die Erfindung soll die Aufgabe gelöst werden, ein Blut-Luft-Separationssystem zu schaffen, welches einen kleineren traumatischen Einfluß auf Blut hat und mit welchem auch mikro-kleine Luftbläschen aus einem fließenden Blutstrom ausgeschieden werden können.

Die Erfindung ist besonders von Vorteil bei Operationen mit Herz-Lungen-Maschinen, Lebertransplantationen, vielen anderen Operationen in Körperkavitäten, und allgemein bei Blutspenden mit einem Blut-Oxygenator.

Ferner soll das System gemäß der Erfindung so ausgebildet sein, daß im abgesaugten Blut vorhandene Luft kurz nach der Blutabsaugstelle, insbesondere nahe beim Patienten wieder vom Blut getrennt werden kann. Das System soll preiswert und leicht zu benutzen sein. Das System der Erfindung soll folgendes ermögichen:
1. Eine maximale Trennung der Gasphase (Luft), auch wenn sie aus kleinen Luftbläschen mit nur wenigen µm Durchmesser besteht, von der Flüssigkeitsphase (Blut), vorzugsweise sofort und direkt an oder nahe an der Stelle, wo das Blut abgesaugt wird;
2. eine Reduzierung des für die Absaugung erforderlichen Vakuums.

Diese Aufgabe wird gemäß der Erfindung durch die unabhängigen Ansprüche gelöst.

Gemäß der Erfindung wird das Blut in einem Zyklon in Wirbelbewegung versetzt, so daß die schwereren Bestandteile des Blut-Luft-Gemisches durch Fliehkraft radial nach außen gedrängt werden, während die physikalisch leichteren Bestandteile und damit insbesondere die Luft in das radiale Zentrum des Zyklon-Wirbelstromes gedrängt werden. Durch getrenntes Absaugen der radial nach außen gedrängten Flüssigkeitsphase und der sich radial innerhalb von ihr bildenden Gasphase wird die Gasphase von der Flüssigkeitsphase getrennt.

In der vorliegenden Beschreibung und den Zeichnungen sind nur Ausführungsformen mit einem Zyklon dargestellt. Für den Fachmann ist es jedoch klar, daß auch mehrere Zyklone parallel oder in Serie benutzt werden können. Die folgende Beschreibung und Darstellung eines Zyklons ist deshalb stellvertretend für Ausführungsformen mit mehreren parallel oder in Reihe verwendeten Zyklonen.

Bei einer bevorzugten Ausführungsform der Erfindung wird der Zyklon in einem kleinen Handgriffteil einer Blutabsaugkanüle untergebracht. Dies hat die Vorteile, daß die Luft unmittelbar nach der Blutabsaugstelle wieder vom Blut getrennt wird, und daß sich der Zyklon an einer gewichtsmäßig günstigen Position befindet. Der Zyklon kann in die Blutabsaugkanüle integriert oder am stromabwärtigen Ende der Blutabsaugkanüle angeordnet sein. Die Außenwand der Kanüle und/oder des Handgriffteils bildet vorzugsweise gleichzeitig die Mantelwand des Zyklons.

Weitere Merkmale der Erfindung sind in den Unteransprüchen enthalten.

Die Erfindung wird im folgenden mit Bezug auf die Zeichnungen anhand von mehreren bevorzugten Ausführungsformen als Beispiele beschrieben. In den Zeichnungen zeigen
- Fig. 1: einen axialen Längsschnitt durch eine Blutabsaugvorrichtung nach der Erfindung im Maßstab 4:1,
- Fig. 2: einen Querschnitt längs der Ebene II-II von Fig. 1,
- Fig. 3: einen Querschnitt längs der Ebene III-III von Fig. 1,
- Fig. 4: eine Seitenansicht eines Strömungskörpers der Blutabsaugvorrichtung von Fig. 1,
- Fig. 5: schematisch die Anwendung einer Blutabsaugvorrichtung nach Fig. 1 in einem System zur Blutabsaugung von einem Patienten, Blutaufbereitung und Blut-Rezirkulation zurück zu dem Patienten,
- Fig. 6: schematisch eine weitere Ausführungsform der Blutabsaugvorrichtung nach der Erfindung,
- Fig. 7: schematisch einen Axialschnitt einer weiteren Ausführungsform eines Zyklons nach der Erfindung, ähnlich der Fig. 1,
- Fig. 8: schematisch einen Axialschnitt einer weiteren Ausführungsform eines Zyklons nach der Erfindung,
- Fig. 9: schematisch einen Axialschnitt einer weiteren Ausführungsform eines Zyklons zur Trennung von Luft von einem fließenden Blutstrom nach der Erfindung.
- Fig. 10: schematisch eine Vorrichtung nach der Erfindung zum Trennen von Luft aus einem fließenden Blutstrom sowohl im Vorlauf als auch im Rücklauf zwischen einem Patienten und einer Blutaufbereitungsvorrichtung, beispielsweise einer Herz-Lungen-Maschine, die einen Oxygenator zur Anreicherung des Blutes mit Sauerstoff aufweist.

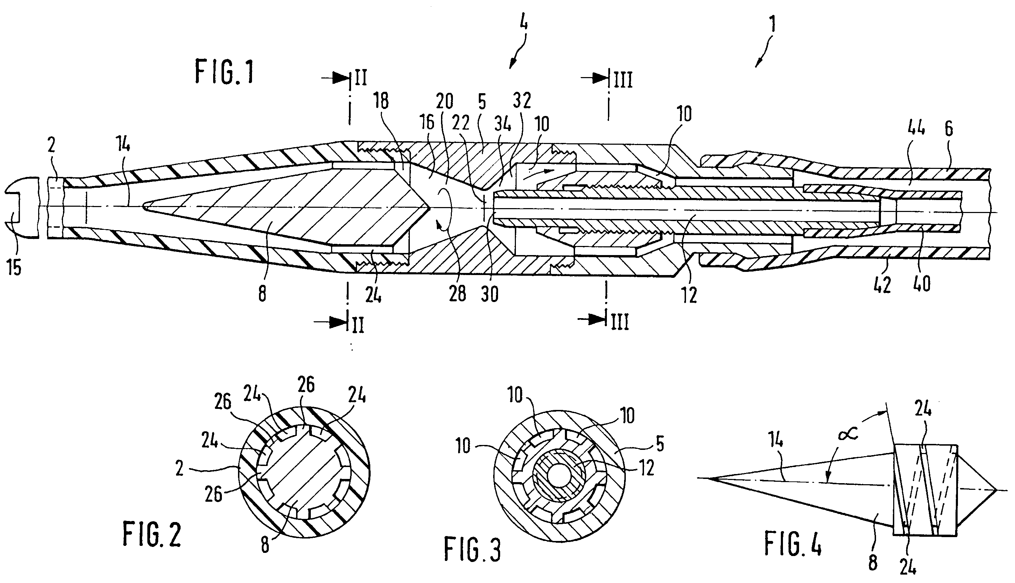

Die Blutabsaugvorrichtung 1 nach den Fig. 1 bis 4 besteht aus einer Blutabsaugkanüle 2, einer Zyklonvorrichtung 4 am stromabwärtigen Ende der Blutabsaugkanüle 2, und einem Doppelschlauch oder Doppellumen 6 am stromabwärtigen Ende der Zyklonvorrichtung 4. Die Zyklonvorrichtung 4 enthält in einer Mantelwand 5 einen Strömungsleitkörper 8, einen im Querschnitt ringförmigen Flüssigkeitskanal 10 und einen darin im radialen Zentrum axial angeordneten Gaskanal 12. Alle Teile sind axial zu einer Mittelachse 14 angeordnet. Die Zyklon-Mantelwand 5 bildet einen Handgriff der Blutabsaugkanüle 2 und kann aus einem Stück mit der Blutabsaugkanüle 2 oder, entsprechend den Zeichnungen, aus mehreren lösbar miteinander verbundenen Teilen bestehen. Die Blutabsaugkanüle 2 hat an ihrem stromaufwärtigen Ende einen Saugeinlaß 15. Die Mantelwand 5 bildet eine Zyklon-Wirbelkammer 16, welche aus einem stromaufwärtigen zylindrischen Abschnitt 18 und einem sich daran anschließenden, in Strömungsrichtung trichterförmig verengenden Düsenabschnitt 20 mit einer Düsenöffnung 22 im radialen Zentrum besteht. Gewindeförmige Nuten 24 zwischen gewindeförmigen Rippen 26 des Strömungsleitkörpers 8 und der an ihnen anliegenden Zyklon-Mantelwand 5 bilden einen "ungefähr tangentialen" Zykloneinlaß am stromaufwärtigen Anfang der Zyklon-Wirbelkammer 16. Der Ausdruck "ungefähr tangential" bedeutet hierin eine Richtung, welche exakt in Tangentialrichtung 90° zur Mittelachse 14 oder mindestens derart schräg zur Mittelachse 14 verläuft, daß der axiale Blut-Luft-Gemisch-Saugstrom der Blutabsaugkanüle 2 in der Zyklon-Wirbelkammer 16 als Wirbelstrom 28 in Umfangsrichtung an der Zyklon-Mantelwand 5 entlangströmt und dadurch Fliehkräfte entstehen, welche die Blutbestandteile (Flüssigkeitsphase) des Gemisch-Saugstromes radial nach außen zur Mantelwand 5 treiben und dadurch von der radial nach innen verdrängten Luft (Gasphase) des Gemisch-Saugstromes trennen. Der trichterartig enger werdende Düsenabschnitt 20 bewirkt eine Verkleinerung des verfügbaren Strömungsquerschnitts und damit eine Erhöhung der Strömungsgeschwindigkeit des Saugstromes in Umfangsrichtung.

Der Gaseinlaß 30 des Gaskanals 12 bildet den Gasauslaß der Zyklon-Wirbelkammer 16 und hat einen kleineren Querschnitt als die Düsenöffnung 22 und befindet sich mit nur kurzem Abstand stromabwärts von dieser Düsenöffnung 22. Der Flüssigkeitseinlaß 32 des Flüssigkeitskanals 10 bildet den Blutauslaß der Zyklon-Wirbelkammer 16 und ist ringförmig zwischen dem Gaseinlaß 30 des Gaskanals 12 und einem sich in Strömungsrichtung im Querschnitt trichterartig erweiternden Diffusor-Kanalabschnitt 34 gebildet, der auf die Düsenöffnung 22 folgt. Der Gaskanal 12 und sein Gaseinlaß 30 bestehen aus einem Rohr, welches in die Zyklon-Mantelwand 5 austauschbar eingesetzt ist. Der trichterartig enger werdende Zyklon-Düsenabschnitt 20 bewirkt eine Strömungsbeschleunigung, und der sich daran anschließende trichterartig weiter werdende Diffusor-Kanalabschnitt 34 bewirkt eine Strömungsverlangsamung der Zyklon-Wirbelströmung. Durch diese Kombination ergibt sich ein besserer Wirkungsgrad der Luft-Blut-Trennung.

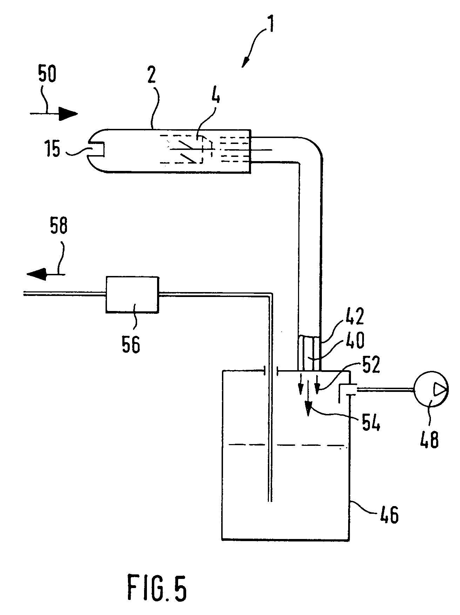

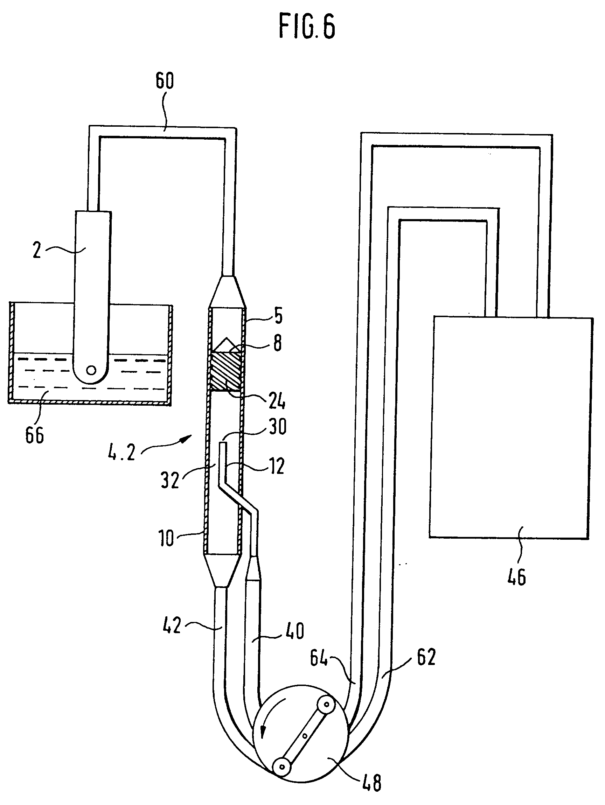

Der Doppelschlauch 6 besteht aus einem radial inneren Schlauch 40, welcher an das stromabwärtige Ende des Rohres des Gaskanals 12 angeschlossen ist, und aus einem radial äußeren Schlauch 42, welcher den inneren Schlauch 40 mit radialem Abstand umgibt und an das stromabwärtige Ende der Zyklon-Mantelwand 5 derart angeschlossen ist, daß der Flüssigkeitskanal 10 mit dem Zwischenraum 44 in Strömungsverbindung steht, welcher zwischen den beiden Schläuchen 40 und 42 gebildet ist. Beide Schläuche 40 und 42 sind an ihren stromabwärtigen, nicht dargestellten Enden entweder gemäß Fig. 6 direkt oder gemäß Fig. 5 unter Zwischenschaltung eines Blutreservoirs 46 an eine Saugquelle 48 angeschlossen.

Die Nuten 24 des Strömungsleitkörpers können einen sich ändernden Steigungswinkel Alpha von ungefähr null Grad am stromaufwärtigen Anfang bis ungefähr neunzig Grad am stromabwärtigen Ende haben, mit Bezug auf die Mittelachse 14 entsprechend Fig. 4.

Fig. 5 zeigt schematisch die Blutabsaugvorrichtung 1, welche in Richtung eines Pfeiles 50 Blut und Luft von einer Wundstelle, beispielsweise einer Operationsstelle, durch den Saugeinlaß 15 der Blutabsaugkanüle 2 absaugt, den Blut-Luft-Gemisch-Saugstrom in der Zyklonvorrichtung 4 in eine Blutphase 52 und eine Gasphase 54 trennt, und beide Phasen 52 und 54 in das Blutreservoir 46 saugt. An das Blutreservoir 46 ist oberhalb seines Flüssigkeitsspiegels die Saugquelle 48 angeschlossen. Das Blut kann vom Blutreservoir 46 über eine Blutaufbereitungs- und Blutfördervorrichtung 56 in Richtung eines Pfeiles 58 wieder in den Kreislauf des Patienten zurück rezierkuliert werden oder in Blutkonserven gefüllt werden.

Bei der Ausführungsform nach Fig. 6 ist die Saugquelle 48 eine Rollerpumpe, welche im Strömungsweg zwischen einer weiteren Ausführungsform einer Zyklonvorrichtung 4.2 und dem Blutreservoir 46 angeordnet ist. Die Blutabsaugkanüle 2 kann bogenförmig oder über einen kurzen Verbindungsschlauch 60 an das stromaufwärtige Ende der Zyklonvorrichtung 4.2 angeschlossen. Das stromabwärtige Ende der Zyklonvorrichtung 4.2 ist durch die beiden Schläuche 40 und 42 an die Saugseite der Rollerpumpe 48 angeschlossen sein. Die Druckseite der Rollerpumpe 48 ist über einen Schlauch 62 für die Flüssigkeitsphase (Blut) und einen separaten Schlauch 64 für die Gasphase (Luft) an das Blutreservoir 46 angeschlossen. Der Patient 66 ist nur schematisch in Form eines Blutgefäßes dargestellt.

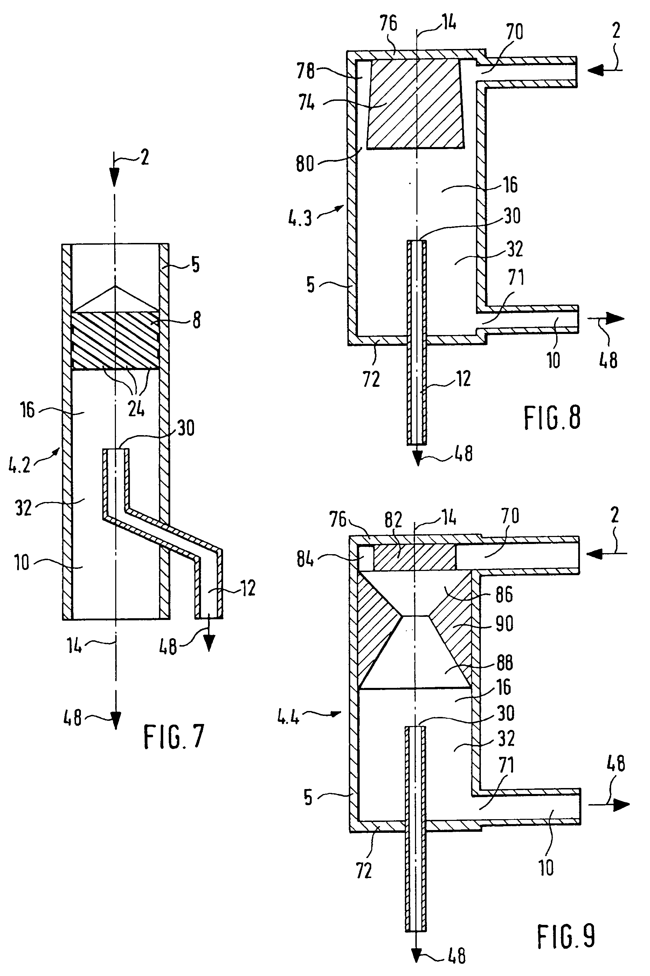

Die in Fig. 7 dargestellte Zyklonvorrichtung 4.2 von Fig. 6 enthält einen Strömungsleitkörper 8, dessen stromaufwärtige Stirnseite die Form eines Kegels mit kurzer Kegelhöhe hat und dessen stromabwärtige Stirnseite flach ist. Die Zyklon-Wirbelkammer 16 hat auf ihrer gesamten axialen Länge eine kreiszylindrische Form. Der Gaseinlaß 30 des Gaskanals 12 ist im radialen Zentrum des vom Strömungsleitkörper 8 erzeugten Wirbelstromes 28 angeordnet. Der Gaskanal 12 ist stromabwärts seines Einlaßes 30 aus der Zyklon-Wirbelkammer 16 seitlich herausgeführt, so daß bei dieser Ausführungsform die beiden Schläuche 40 und 42 nicht koaxial ineinander, sondern außerhalb voneinander liegen.

Bei den vorstehend beschriebenen Ausführungsformen erstreckt sich die Mittelachse 14 in der Zyklonvorrichtung 4 oder 4.2, je nach Position der Bedienungsperson horizontal, vertikal oder schräg.

Die in den Fig. 8 und 9 dargestellten Ausführungsformen von Zyklonvorrichtungen 4.3 und 4.4 können als selbständige Geräte auf eine Unterlage gestellt werden, so daß die Mittelachse 14 der Zyklonvorrichtungen 4.3 und 4.4 sich vertikal erstreckt. Die Zyklonvorrichtung 4.3 von Fig. 8 und die Zyklonvorrichtung 4.4 von Fig. 9 haben je eine kreiszylindrische Zyklon-Mantelwand 5; am oberen, stromaufwärtigen Ende der Mantelwand 5 einen tangentialen Zykloneinlaß 70, welcher an eine nicht dargestellte Blutabsaugkanüle 2 angeschlossen ist; am unteren, stromabwärtigen Ende der Mantelwand 5 einen tangentialen ersten Zyklonauslaß 71, welcher ein Teil des Flüssigkeitskanals 10 ist, dessen Einlaß 32 durch den Ringraum zwischen der Zyklon-Mantelwand 5 und dem Gaskanal 10 gebildet ist; einen zweiten Zyklonauslaß in Form des Einlasses 30 des Gaskanals 12, welcher sich durch einen Wirbelkammerboden 72 hindurch erstreckt, so daß der Einlaß 30 des Gaskanals 12 im radialen Zyklonzentrum der Wirbelkammer 16 stromaufwärts von dem Flüssigkeits-Einlaß 32 des Flüssigkeitskanals angeordnet ist.

Bei der Ausführungsform nach Fig. 8 befindet sich in der Zyklon-Wirbelkammer 16 ein kegelförmiger Einsatzkörper 74, welcher sich von einer Wirbelkammer-Decke 76 oberhalb des Zykloneinlasses 70 am Zykloneinlaß 70 vorbei erstreckt und zwischen sich und der Zyklon-Mantelwand 5 einen ringförmigen Düsenkanal 78 konzentrisch zur Mittelachse 14 bildet, der sich vom Zykloneinlaß 70 bis zu einer ringförmigen Düsenöffnung 80 keilförmig verengt und dadurch den Blut-Luft-Gemisch-Saugstrom beschleunigt. Die stromabwärtige Stirnseite des Einsatzkörpers 74 ist rechtwinkelig zur Mittelachse 14 flach und liegt mit Abstand gegenüber von dem Einlaß 30 des Gaskanals 12.

Die Zyklonvorrichtung 4.4 von Fig. 9 hat auf der Innenseite ihrer Wirbelkammer-Decke 76 einen kreiszylindrischen Einsatzkörper 82. Der Einsatzkörper 82 bildet mit der Zyklon-Mantelwand 5 im Bereich des Zykloneinlasses 70 eine Ringkammer 84. Auf die Ringkammer 84 folgt in Strömungsrichtung längs der Mittelachse 14 ein trichterartig enger werdender Düsenabschnitt 86 und dann ein trichterartig weiter werdender Diffusorabschnitt 88, welch letzterem mit axialem Abstand der Einlaß 30 des Gaskanals 12 axial gegenüberliegt. Der Düsenabschnitt 86 und der Diffusorabschnitt 88 sind durch einen zweiten Einsatzkörper 90 gebildet, welcher in die Zyklon-Mantelwand 5 eingesetzt ist.

Die Zyklonvorrichtungen nach der Erfindung bilden einen "dynamischen Luftabscheider", weil er vom Blut durchströmt wird und dabei Luft aus dem Blut abscheidet. Die Zyklonvorrichtungen haben auch bei sehr kleinen Blutmengen und bei sehr kleinen, nur wenige µm großen Luftbläschen noch einen guten Luftabscheidungs-Wirkungsgrad.

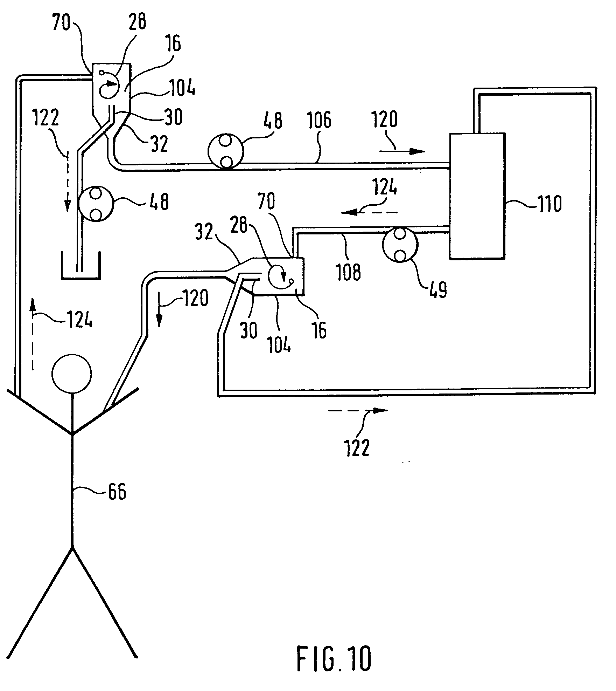

Die in Fig. 10 dargestellte Vorrichtung nach der Erfindung enthält einen Zyklon 104 im Vorlauf 106 und einen Zyklon 104 im Rücklauf 108 eines Blutkreislaufes von einem Patienten 66 zu einer Herz-Lungen-Maschine 110, die einen Oxygenator zur Sauerstoffanreicherung des Blutes enthält, und wieder zurück zum Patienten 66. Zur Absaugung des Blutes vom Patienten 66 befindet sich im Vorlauf 106 zwischen seinem Zyklon 104 und der Herz-Lungen-Maschine 110 eine Pumpe 48. Die Gasphase kann aus dem Zyklon 104 des Vorlaufes 106 durch einen separaten Strömungsweg der gleichen Pumpe oder durch eine zweite Pumpe 48 abgesaugt werden.

Im Rücklauf 108 ist zwischen der Herz-Lungen-Maschine 110 und ihrem Zyklon 104 eine Pumpe 49 angeordnet. In diesem Fall wird das Blut nicht durch diesen Zyklon 104 hindurchgesaugt, sondern hindurchgetrieben. Die Gasphase dieses Zyklons 104 im Rücklauf 108 kann durch die Förderkraft seiner Pumpe 104 in die Herz-Lungen-Maschine 110 zurückgeleitet werden.

Die Zyklone 104 des Vorlaufs 106 und des Rücklaufs 108 haben am einen axialen Ende einer kreisrunden Zyklon-Wirbelkammer 16 einen tangentialen Einlaß 70 und an ihrem anderen axialen Ende einen Blutauslaß 32 an der Zyklonenkammerwand und einen Luftauslaß 30 im radialen Zentrum der Zyklonen-Wirbelkammer 16.

Der Vorlauf 106 und der Rücklauf 108 bilden zwei verschiedene Prozesse, welche getrennt voneinander oder gemäß Fig. 10 in Kombination miteinander verwendet werden können.Der Zyklon 104 im Vorlauf 106 dient zum Trennen von Luft, welche unerwünscht am Patient abgesaugt wird, von dem Blut, welches gleichzeitig vom Patienten abgesaugt wird. Der Zyklon 104 im Rücklauf 108 dient zum Trennen von kleinsten Luftbläschen mit einem Durchmesser im Bereich von µm, welche im Oxygenerator der Maschine 110 in das Blut gelangen, das zum Patienten gefördert wird.

Gemäß einer nicht dargestellten Ausführungsform kann die Pumpe 48 im Vorlauf weggelassen werden und ihre Saugwirkung von der Pumpe 49 des Rücklaufs 108 durch die Maschine 110 hindurch erzeugt werden. In Fig. 10 geben ausgezogene Pfeile 120 die Blut-Strömungsrichtung, gestrichelte Pfeile 122 die Luft-Strömungsrichtung, und kombiniert ausgezogen-gestrichelte Pfeile 124 die Blut-Luft-Gemisch-Strömungsrichtung an.

Die Blutabsaugstelle kann eine Wundstelle, Qperationsstelle oder jedes beliebige Blutgefäß eines Patienten 66 sein, oder ein Behälter, zum Beispiel eine Blutkonserve, oder eine Maschine, zum Beispiel eine Herz-Lungen-Maschine 110, und/oder ein Oxygenator zur Sauerstoffanreicherung von Blut.

## Patentansprüche

1. Vorrichtung zum Ausscheiden von Luft aus lufthaltigem Blut, mit folgenden Merkmalen: eine Zyklonvorrichtung (4;4.2;4.3;4.4;104) mit einer Zyklon-Wirbelkammer (16), durch welche das lufthaltige Blut als fließender Blutstrom in Form einer rotierenden Zyklonströmung hindurchführbar ist, so daß in der rotierenden Zyklonströmung Fliehkräfte zur Trennung der Luft vom Blut entstehen; Zyklonauslässe (30,32), durch welche vom radial äußeren Umfangsbereich der Zyklonströmung einerseits und vom radial inneren Bereich der Zyklonströmung andererseits Bestandteile des lufthaltigen Blutes getrennt aus der Zyklonströmung herausführbar sind, wobei wegen der Fliehkräfte in der rotierenden Zyklonströmung die aus dem Umfangsbereich der Zyklonströmung herausgeführten Bestandteile volumenmäßig weniger Luft enthalten, während die aus dem radialen inneren Bereich der Zyklonströmung herausgeführten Bestandteile entsprechend volumenmäßig mehr Luft enthalten als das lufthaltige Blut stromaufwärts der Zyklonströmung; die Zyklon-Wirbelkammer (16) hat an einem axialen Ende einen Zykloneinlaß (24;70) für das lufthaltige Blut und am anderen axialen Ende den Zyklonauslaß (32) für die Blutphase an der Mantelwand der Zyklon-Wirbelkammer (16); dadurch gekennzeichnet, daß die Zyklon-Wirbelkammer (16) an dem genannten anderen Ende auch einen Zyklonauslaß (30) für die Gasphase hat, welcher im radial inneren Zentrum der Zyklonströmung angeordnet ist.

2. Vorrichtung nach Anspruch 1,
**dadurch gekennzeichnet,**
daß der Zyklonauslaß (30) für die Gasphase koaxial innerhalb des Zyklonauslasses (32) für die Blutphase angeordnet ist.

3. Vorrichtung nach einem der Ansprüche 1 oder 2,
**dadurch gekennzeichnet,**
daß die Zyklon-Wirbelkammer (16) einen stromabwärts in Richtung zu ihrem Zyklonauslaß (32) für die Blutphase düsenartig enger werdenden Kammerabschnitt (20;78;86) hat.

4. Vorrichtung nach einem der Ansprüche 1 bis 3,
**dadurch gekennzeichnet,**
daß die Zyklonvorrichtung (4;4.2;4.3;4.4;104) im Strömungsweg zwischen einer Blutabsaugstelle (66;110) und einer Saugquelle (48) liegt, wobei sie wesentlich näher bei der Absaugstelle als bei der Saugquelle (48) angeordnet ist, durch welche das Blut von der Blutabsaugstelle absaugbar ist.

5. Vorrichtung nach Anspruch 4,
**dadurch gekennzeichnet,**
daß die Zyklonvorrichtung (4) in einem Handgriffteil (5) einer Blutabsaugkanüle (2) integriert ist.

6. Vorrichtung nach einem der Ansprüche 1 bis 5,
**dadurch gekennzeichnet,**
daß die Zyklonvorrichtung (4;4.2;4.3;4.4;104) in einem Blut-Rezirkulationskreislauf (4,46,56;106,108,110) angeordnet ist.

7. Vorrichtung nach einem der Ansprüche 1 bis 6,
**dadurch gekennzeichnet,**
daß die Zyklonvorrichtung (49) in einem Blutströmungsweg (56;108) angeordnet ist, der von einer Blutspendervorrichtung (46;110) zu einem Blutempfänger führt.

8. Vorrichtung nach einem der Ansprüche 3 bis 7,
**dadurch gekennzeichnet,**
daß dem in Strömungsrichtung düsenartig enger werdenden Zyklon-Wirbelkammerabschnitt (20;86) ein in Strömungsrichtung trichterartig weiter werdender Zyklon-Wirbelkammer-Diffusorabschnitt (34; 88) nachgeordnet ist, welcher einen Teil des Zyklonauslasses (32) für die Blutphase bildet.

9. Vorrichtung nach einem der Ansprüche 1 bis 8,
**dadurch gekennzeichnet,**
daß der Zykloneinlaß (70) einen sich keilförmig verengenden Abschnitt aufweist, welcher das lufthaltige Blut beschleunigt, das in die Zyklon-Wirbelkammer (16) strömt.

10. Vorrichtung nach einem der Ansprüche 1 bis 9,
**dadurch gekennzeichnet,**
daß der Zykloneinlaß (20) gewindeförmige Nuten (24) zwischen gewindeförmigen Rippen (26) aufweist, durch welche aus einer axialen Strömungsrichtung des lufthaltigen Blutes eine ungefähr tangentiale Richtung am stromaufwärtigen Anfang der Zyklon-Wirbelkammer (16) gebildet wird.

## Claims

1. A device for eliminating air from blood containing air, having the following features:
a cyclone device (4; 4.2; 4.3; 4.4; 104) with a cyclone eddy chamber (16), through which the blood containing air can be guided as a flowing blood flow in the form of a rotating cyclone flow, so that centrifugal forces are generated in the rotating cyclone flow for separating the air from the blood; cyclone outlets (30, 32), through which components of the blood containing air can exit separately from the radially outer circumferential region of the cyclone flow on the one hand and from the radially inner region of the cyclone flow on the other hand, wherein, on account of the centrifugal forces in the rotating cyclone flow, the components guided out of the circumferential region of the cyclone flow contain less air per unit volume, whilst the components guided out of the radially inner region of the cyclone flow accordingly contain more air per unit volume than the blood containing air upstream of the cyclone flow; the cyclone eddy chamber (16) comprises, at one axial end, a cyclone inlet (24; 70) for the blood containing air and at its other axial end the cyclone outlet (32) for the blood phase on the circumferential wall of the cyclone eddy chamber (16); characterised in that the cyclone eddy chamber (16) at the said other end also comprises a cyclone outlet (30) for the gas phase, which is arranged in the radially inner centre of the cyclone flow.

2. A device according to claim 1, characterised in that the cyclone outlet (30) for the gas phase is arranged coaxially within the cyclone outlet (32) for the blood phase.

3. A device according to one of claims 1 or 2, characterised in that the cyclone eddy chamber (16) comprises a chamber section (20; 78; 86) tapering in the manner of a nozzle downstream in the direction of its cyclone outlet (32) for the blood phase.

4. A device according to one of claims 1 to 3, characterised in that the cyclone device (4; 4.2; 4.3; 4.4; 104) lies in the flow path between a blood extraction site (66; 110) and a vacuum source (48), and is arranged substantially closer to the extraction site than the vacuum source (48) by means of which the blood can be extracted from the blood extraction site.

5. A device according to claim 4, characterised in that the cyclone device (4) is integrated in a handle element (5) of a blood extracting canula (2).

6. A device according to one of claims 1 to 5, characterised in that the cyclone device (4; 4.2; 4.3; 4.4; 104) is arranged in a blood recirculation circuit (4, 46, 56; 106, 108, 110).

7. A device according to one of claims 1 to 6, characterised in that the cyclone device (49) is arranged in a blood flow path (56; 108), which leads from a blood dispensing device (46; 110) to a blood receiver.

8. A device according to one of claims 3 to 7, characterised in that, arranged downstream of the cyclone eddy chamber section (20; 86) tapering in the manner of a nozzle in the direction of flow, is a cyclone eddy chamber diffuser section (34; 88) widening in the manner of a funnel in the direction of flow, which forms part of the cyclone outlet (32) for the blood phase.

9. A device according to one of claims 1 to 8, characterised in that the cyclone outlet (70) comprises a section tapering in the manner of a wedge, which accelerates the blood containing air flowing into the cyclone eddy chamber (16).

10. A device according to one of claims 1 to 9, characterised in that the cyclone inlet (20) comprises thread-like grooves (24) between thread-like ribs (26), by means of which, from an axial flow direction of the blood containing air, an approximately tangential direction is formed at the upstream start of the cyclone eddy chamber (16).

## Revendications

1. Dispositif pour séparer l'air du sang chargé en air, présentant les caractéristiques suivantes :
un dispositif séparateur à cyclone (4 ; 4.2 ; 4.3 ; 4.4 ; 104) équipé d'une chambre tourbillonnaire 16 du séparateur à cyclone, par laquelle le sang chargé en air peut traverser en tant que flux sanguin en écoulement sous la forme d'un courant tourbillonnaire rotatif, de sorte que se forment dans le courant tourbillonnaire rotatif des forces centrifuges permettant de séparer l'air du sang ; des sorties du séparateur à cyclone (30, 32), par lesquelles, depuis la zone périphérique externe radiale du courant tourbillonnaire d'une part et depuis la zone interne radiale du courant tourbillonnaire d'autre part, les composants du sang chargé en air peuvent être amenés séparément hors du courant tourbillonnaire, où, en raison des forces centrifuges dans le courant tourbillonnaire rotatif, les composants sanguins sortant de la zone périphérique du courant tourbillonnaire contiennent moins d'air en volume, tandis que les composants sortant de la zone interne radiale du courant tourbillonnaire contiennent de façon correspondante un volume d'air plus important que le sang chargé en air en amont du courant tourbillonnaire ; la chambre tourbillonnaire (16) du séparateur à cyclone présente, au niveau d'une extrémité axiale, une entrée de séparateur à cyclone (24 ; 70) pour le sang chargé en air et, au niveau de l'autre extrémité axiale, la sortie du séparateur à cyclone (32) pour la phase sanguine au niveau de la paroi enveloppante de la chambre tourbillonnaire (16) du séparateur à cyclone ; caractérisé en ce que la chambre tourbillonnaire (16) du séparateur à cyclone présente également à ladite autre extrémité une sortie de séparateur à cyclone (30) pour la phase gazeuse, laquelle est disposée dans le centre interne radial du courant tourbillonnaire.

2. Dispositif selon la revendication 1, caractérisé en ce que la sortie du séparateur à cyclone (30) pour la phase gazeuse est disposée de façon coaxiale à l'intérieur de la sortie du séparateur à cyclone (32) pour la phase sanguine.

3. Dispositif selon l'une des revendications 1 ou 2, caractérisé en ce que la chambre tourbillonnaire du séparateur à cyclone (16) présente un segment de chambre se rétrécissant en forme de buse (20 ; 78 ; 86) en aval dans la direction de la sortie du séparateur à cyclone (32) pour la phase sanguine.

4. Dispositif selon l'une des revendications 1 à 3, caractérisé en ce que le dispositif séparateur à cyclone (4 ; 4.2 ; 4.3 ; 4.4 ; 104) est disposé sur le circuit d'écoulement entre un emplacement d'aspiration du sang (66 ; 110) et une source d'aspiration (48), celui-ci étant disposé essentiellement plus près de l'emplacement d'aspiration que de la source d'aspiration (48), par laquelle le sang peut être aspiré depuis la source d'aspiration du sang.

5. Dispositif selon la revendication 4, caractérisé en ce que le dispositif séparateur à cyclone (4) est intégré dans une portion de poignée (5) d'une canule d'aspiration du sang (2).

6. Dispositif selon l'une des revendications 1 à 5, caractérisé en ce que le dispositif séparateur à cyclone (4 ; 4.2 ; 4.3 ; 4.4 ; 104) est disposé dans un circuit de remise en circulation du sang (4, 46, 56 ; 106, 108, 110).

7. Dispositif selon l'une des revendications 1 à 6, caractérisé en ce que le dispositif séparateur à cyclone (49) est disposé dans un circuit d'écoulement du sang (56 ; 108) qui est acheminé depuis un dispositif de prélèvement de sang (46 ; 110) vers un receveur de sang.

8. Dispositif selon l'une des revendications 3 à 7, caractérisé en ce qu'un segment diffuseur (34 ; 88) de la chambre tourbillonnaire du séparateur à cyclone s'élargissant en entonnoir dans la direction de l'écoulement est subordonné à un segment (20 ; 86) de la chambre tourbillonnaire du séparateur à cyclone se rétrécissant en forme de buse dans la direction de l'écoulement, lequel segment diffuseur forme une portion de la sortie du séparateur à cyclone (32) pour la phase sanguine.

9. Dispositif selon l'une des revendications 1 à 8, caractérisé en ce que l'entrée du séparateur à cyclone (70) présente un segment se rétrécissant en coin, lequel accélère le sang chargé en air qui s'écoule dans la chambre tourbillonnaire (16) du séparateur à cyclone.

10. Dispositif selon l'une des revendications 1 à 9, caractérisé en ce que l'entrée du séparateur à cyclone (20) présente des rainures en filets (24) entre des nervures en filets (26), par lesquelles depuis une direction d'écoulement axiale du sang chargé en air est formée une direction approximativement tangentielle au niveau du début en amont de la chambre tourbillonnaire (16) du séparateur à cyclone.
